# EUROPEAN PATENT SPECIFICATION

(11) **EP 3 191 603 B1**
(45) Date of publication and mention of the grant of the patent: **31.10.2018**
(21) Application number: 15766213.1
(22) Date of filing: 11.09.2015
(51) Int. Cl.: C12Q 1/68

(54) **METHOD OF OBTAINING PAIRED-END SEQUENCING INFORMATION**
VERFAHREN ZUR GEWINNUNG VON PAIRED-END-SEQUENZIERUNGSINFORMATIONEN
PROCÉDÉ D'OBTENTION D'INFORMATIONS DE SÉQUENÇAGE D'EXTRÉMITÉS APPARIÉES

(30) Priority: 11.09.2014 GB 201416106
(43) Date of publication of application: 19.07.2017
(73) Proprietor: Illumina Cambridge Limited, Little Chesterford Saffron Walden Essex CB10 1XL (GB)
(72) Inventor: RIGATTI, Roberto, Saffron Walden Essex CB10 1XL (GB); BOUTELL, Jonathan, Saffron Walden Essex CB10 1XL (GB)
(74) Representative: Murgitroyd & Company
(86) International application number: PCT/GB2015/052626
(87) International publication number: WO 2016/038381

(56) References cited:
- WO-A2-2007/086935
- WO-A2-2013/012440
- MING XU ET AL: "Dual primer emulsion PCR for next- generation DNA sequencing", BIOTECHNIQUES, vol. 48, no. 5, 1 May 2010 (2010-05-01), pages 409-412, XP055227583, US ISSN: 0736-6205, DOI: 10.2144/000113423
- BRENT C. SATTERFIELD: "Cooperative Primers", JOURNAL OF MOLECULAR DIAGNOSTICS,THE, vol. 16, no. 2, 1 March 2014 (2014-03-01), pages 163-173, XP055417313, US ISSN: 1525-1578, DOI: 10.1016/j.jmoldx.2013.10.004

## Description

The invention relates generally to the field of nucleic acid sequencing. In particular, the invention relates to a method of obtaining paired-end sequencing information. The invention also relates to a kit for use in the method and a system incorporating the method.

Paired-end (PE) or pairwise sequencing is a known technique involving sequencing both ends of the nucleic acid fragments in a sequencing library and aligning the forward and reverse reads as read pairs.

In addition to producing twice the number of reads for the same time and effort in library preparation, sequences aligned as read pairs enable more accurate read alignment.
Paired-end sequencing allows users to sequence both ends of a fragment and generate high-quality, alignable sequence data. It facilitates detection of genomic rearrangements and repetitive sequence elements, as well as gene fusions and novel transcripts. Since paired-end reads are more likely to align to a reference, the quality of the entire data set is improved by using this technique.
It would be desirable to provide an improved method of obtaining paired-end sequencing information in order to reduce cost, increase throughput, and/or simplify the process.

Ming, Y. et al., "Dual Primer Emulsion PCR for Next-Generation DNA Sequencing", Biotechniques, 48(5), p 409, 2010 discloses a method of dual primer emulsion PCR wherein both primers are attached to a bead.

WO 2007/086935 A2 discloses a method of DNA sequencing comprising preparing a sample of DNA, amplifying the prepared DNA and performing multiple sequencing reactions on the amplified DNA with only one primer hybridisation step.

Satterfield, B. et al., "2.5 Million-Fold Improvement in the Reduction of Nonspecific Amplification", the Journal of Molecular Diagnostics, Vol. 16, No. 2, 2014 discloses a method of reducing primer-dimer propagation using cooperative primers.

### Statement of Invention

One aspect of the invention provides a method of obtaining paired-end sequencing information in a sequencing reaction comprising the steps of:
providing a target nucleic acid immobilized on a solid support;
determining the sequence of the target nucleic acid by generating a complementary nucleic acid strand, the complementary nucleic acid strand comprising at least one linkable moiety;
determining the sequence of the complementary nucleic acid strand; and
comparing the sequences of the target nucleic acid and the complementary nucleic acid strand to provide the paired-end sequencing information.

Advantageously, the at least one linkable moiety may be incorporated into the complementary strand during synthesis of the strand.

The at least one linkable moiety may be provided on a first sequencing primer.
The at least one linkable moiety may be provided on an extension oligonucleotide.
The at least one linkable moiety may be provided on a modified nucleotide.

In one embodiment, the solid support comprises at least one corresponding linkable moiety for selectively covalently or non-covalently binding the at least one linkable moiety on the complementary nucleic acid strand under predetermined conditions.

In one embodiment, the at least one corresponding linkable moiety comprises a surface oligonucleotide on the solid support and the at least one linkable moiety comprises a nucleic acid sequence substantially complementary to the surface oligonucleotide.

In one embodiment, the target nucleic acid may be immobilized on the solid support by a surface oligonucleotide comprising a first sequence and the corresponding linkable moiety surface oligonucleotide on the solid support may have a second sequence that differs from the first sequence.

This provides the advantage of minimizing undesirable hybridization during the hybridization step, because different surface oligonucleotides may be utilized for an amplification step and a hybridization step.

In one embodiment, increased temperature in a hybridization step may inhibit hybridization of the linkable moiety to a surface oligonucleotide on the solid support.
This provides the advantage of minimizing undesirable hybridization during the hybridization step.
The invention provides the advantage that the complementary strand may be anchored to a surface primer on a solid support of the type used for next generation sequencing reactions.

In one embodiment, the at least one corresponding linkable moiety comprises a portion of the sequence of the target nucleic acid immobilized on the solid support.
In certain embodiments, a strand displacing polymerase may be used in an extension step.
Optionally, the method comprises the step of activating the at least one linkable moiety to bind the complementary nucleic acid strand to the solid support.

Advantageously, activation may be performed during a sequencing step. For example, by the addition of an activation molecule during a sequencing by synthesis reaction. Alternatively, or in addition, activation may be by changing one or more conditions of the reaction.

In one embodiment, the at least one linkable moiety comprises a biotinylated moiety on the complementary strand and streptavidin on the solid support.

Preferably, the method comprises the step of removing the target nucleic acid following the step of determining its sequence.

Removal of the target nucleic acid may be by a linearization step.

Advantageously, the removal of the target nucleic acid may be performed during a sequencing step. For example, during cycles of sequencing by synthesis.

In some embodiments, only identical nucleic acids are bound to the solid support at any one time. Either the target or the complementary strand is immobilized on the solid support during a sequencing read.

In a preferred embodiment, the step of determining the sequence of the target nucleic acid comprises hybridizing a first sequencing primer to the target nucleic acid and extending the first sequencing primer to obtain a first sequencing read.

Optionally, the first sequencing primer is extended to substantially the full length of the target nucleic acid following the first sequencing read.

Advantageously, the complementary nucleic acid strand may be extended to substantially the full length of the target nucleic acid.

Preferably, the step of determining the sequence of the complementary nucleic acid strand comprises hybridizing a second sequencing primer to the complementary nucleic acid strand and extending the second sequencing primer to obtain a second sequencing read.

The sequencing read may be obtained using sequencing by synthesis or sequencing by ligation.

The step of comparing the sequences of the target nucleic acid and the complementary nucleic acid strand to provide the paired-end sequencing information may utilize an algorithm and/or computer processor.

The target nucleic acid may comprise a single stranded nucleic acid.

In one embodiment, the target nucleic acid may comprise a double stranded nucleic acid.

In one embodiment, the target nucleic acid comprises a nucleic acid that is part single stranded and part double stranded.

The target nucleic acid may be immobilized on the solid support as a double stranded nucleic acid prior to providing single strands for sequencing.

The target nucleic acid may be immobilized on the solid support as a double stranded nucleic acid.

A recombinase may facilitate hybridization of a sequencing primer into the double stranded nucleic acid.

In one embodiment, the method comprises the pre-step of clonal amplification of the target nucleic acid molecule.

Advantageously, the method may be applied to a cluster or clonal population.

The nucleic acid may comprise DNA or RNA.

The solid support may comprise a flow cell or a bead.
Another aspect of the invention provides a primer and a surface attached oligonucleotide for use in the method comprising a sequence for hybridizing to a target nucleic acid sequence and a linkable moiety for immobilizing a nucleic acid strand complementary to a target nucleic acid sequence to a solid support, wherein the linkable moiety comprises a nucleic acid sequence substantially complementary to a surface oligonucleotide on the solid support, and wherein the sequence for hybridizing to a target nucleic acid sequence is attached at its 5' end to the 5' end of the nucleic acid sequence of the linkable moiety.

The sequence for hybridizing to a target nucleic acid sequence is attached at its 5' end to the 3' end of the nucleic acid sequence of the linkable moiety.

Another aspect of the invention provides a system to obtain paired-end sequencing information comprising sequencing apparatus having a solid support for immobilizing a target nucleic acid, an inlet and outlet for sequencing reagents, and
means for determining the sequence of the target nucleic acid by generating a complementary nucleic acid strand, the complementary nucleic acid strand comprising at least one linkable moiety;
determining the sequence of the complementary nucleic acid strand; and
comparing the sequences of the target nucleic acid and the complementary nucleic acid strand to provide the paired-end sequencing information.

In one embodiment the apparatus comprises a streptavidin-coated solid support.

Another aspect of the invention provides a kit to obtain paired-end sequencing information in a sequencing reaction comprising one or more sequencing reagents and the linkable moiety described above.

The kit may comprise instructions for performing the method of the invention.

### Brief Description of the Drawings

In the Figures, which illustrate aspects of the invention by way of example only:
Figures 1A and 1B illustrate side views of a region of a sequencing substrate for high throughput sequencing;
Figures 2A through 2E illustrate side views of the sequencing substrate of Figure 1B and show an example of a process of obtaining paired-end sequencing information when only one type of DNA strand is bound to the surface of a solid support;
Figures 3A through 3E illustrate side views of the sequencing substrate of Figure 1B and show another example of a process of obtaining paired-end sequencing information when only one type of DNA strand is bound to the surface of a solid support; and
Figures 4A through 4E illustrate side views of the sequencing substrate of Figure 1B and show yet another example of a process of obtaining paired-end sequencing information when only one type of DNA strand is bound to the surface of a solid support.
Figures 5A and 5B illustrate a comparison of the steps of the prior art method shown in 5A to the steps of the method of present invention, outlined in Figure 5B.
Figures 6A to 6E illustrate side views of the sequencing substrate of Figure 1B and show an example of a process of obtaining paired-end sequencing information.
Figures 7A and 7B illustrate side views of the sequencing substrate of Figure 1B and show an example of a process of obtaining paired-end sequencing information.

### Description

The invention provides methods of obtaining paired-end sequencing information when only one type of DNA strand is bound to the surface of a solid support. For example, one type of DNA strand may be bound and clonally amplified on a solid support (e.g., a flow cell) using a single type of oligonucleotide capture probe. One type of DNA strand may be designated as "A" and its complementary strand may be designated "A prime" (A'). In various embodiments, one type of DNA strand (e.g., strand A) is bound to the surface of a solid support and used to generate a first sequencing read. Subsequently, a substantially full length complementary strand (e.g., strand A') is generated and bound to the surface of the solid support. The first type of DNA strand (e.g., strand A) is removed and a second sequencing read is obtained from the surface bound complementary strand (e.g., strand A').

In one example, a first sequencing primer that includes a linkable moiety is used to obtain a first sequencing read. After completion of the first sequencing read, full extension of the sequencing primer is performed in the presence of dNTPs (natural or non-natural) and DNA polymerase to generate a substantially full length complementary strand A'. The complementary strand A' is subsequently bound to the surface of the solid support via the linkable moiety in the fully extended first sequencing primer.

In another example, a first sequencing primer that was used to obtain a first sequencing read from strand A is removed and an extension oligonucleotide that includes a linkable moiety is hybridized and used to generate a substantially full length complementary strand A'. The complementary strand A' is then anchored to the surface of the solid support via the linkable moiety.

In yet another example, modified nucleotides that include a linkable moiety may be incorporated during cycles of a first sequencing read. These nucleotides may be incorporated during the first few cycles or can be incorporated during later cycles - particularly if the molecules are much longer than the read length.

After completion of the first sequencing read, full extension of the first sequencing primer is performed in the presence of dNTPs and DNA polymerase to generate a substantially full length complementary strand A'. The complementary strand A' is subsequently bound to the surface of the solid support via the linkable moieties on the incorporated modified nucleotides.

The subsequent binding and/or sequencing of the complementary strand A' may be on the same substrate/support, at substantially the same or different region of the first sequencing read. Alternatively or in addition, it may be on a different substrate/support.

Certain conditions or activation of linkable moieties may be required in order for the linkable moiety associated with the strand A' to bind an associated moiety on the surface of the solid support, such that the anchorage of the complementary strand to the surface can be controlled.

For example, in certain embodiment multiple step chemistries are used such that the linkable moiety does not couple to the solid support moiety until a third moiety or other condition (e.g., pH or temperature change) is added. This permits control and, if desired, deferring of the coupling of the linkable moiety during sequencing. Non-limiting examples of suitable attachment chemistry for use in the invention are described in US application number 13/784,368.

Alternatively or in addition, controlled activation of the linkable moiety or moieties may be achieved by ligand-receptor / antibody-antigen interactions, such as binding of an antibody to an antigen

The methods described herein may be applied to single DNA molecules (e.g., strand A) or to clonal populations of DNA molecules (e.g., clonal populations of strand A) bound to the surface of a solid support.

Therefore, as compared to current paired-end sequencing protocols, the invention for obtaining paired-end sequencing information provide for simpler methods of obtaining paired-end sequencing information.

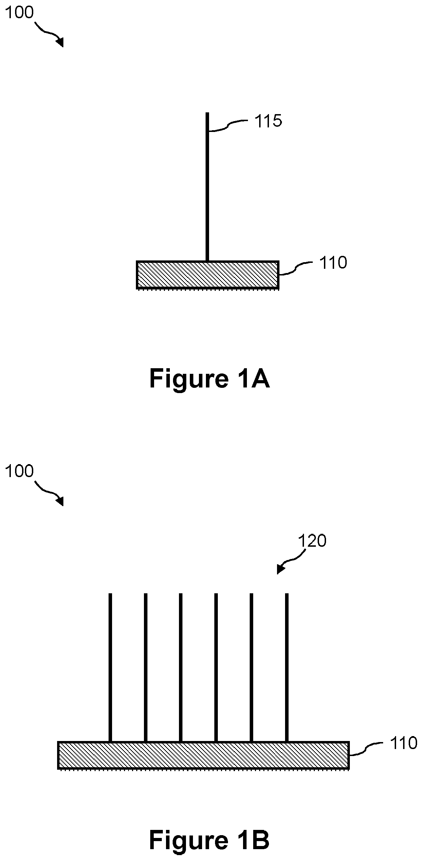

**Figures 1A and 1B** illustrate side views of a region of a sequencing substrate 100 for high throughput sequencing. Sequencing substrate 100 includes a solid support 110. In one example, solid support 110 is a planar substrate, such as a flow cell. In another example, solid support 110 is a bead (not shown). Referring to Figure 1A, a single type of DNA strand 115 may be bound to the surface of solid support 110. DNA strand 115 may be referred to as the A strand. The reverse complement of strand A may be referred to as strand A' (not shown).

Referring to Figure 1B, a clonal population 120 of DNA molecules may be bound to the surface of solid support 110. Clonal population 120 is a population of identical DNA molecules that are derived by clonal amplification of a single DNA molecule that is to be sequenced. All identical copies in clonal population 120 are attached to solid substrate 110. In one example, clonal population 120 comprises one type of DNA strand (e.g., strand A). In another example (not shown), a first clonal population 120 may comprise strand A and a second clonal population 120 may comprise strand A'. In this example, a single clonal population 120 is shown, but any number of clonal populations may be bound to the surface of solid support 110.

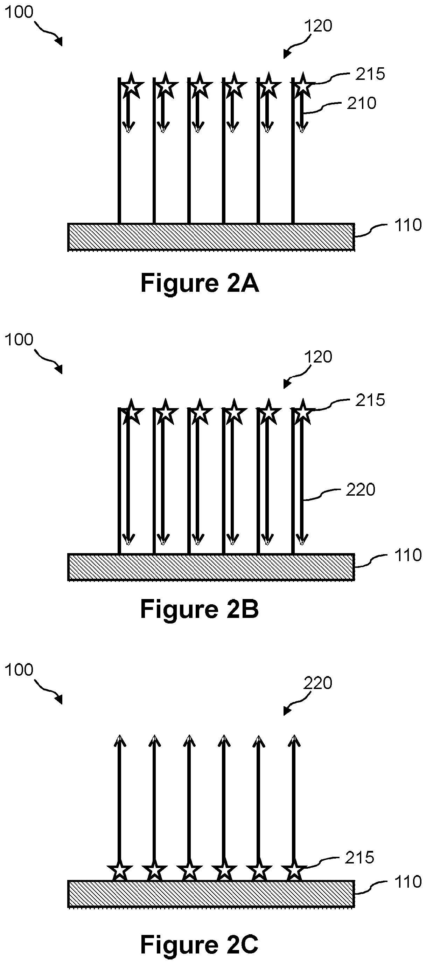

**Figures 2A through 2E** illustrate side views of sequencing substrate 100 of Figure 1B and show an example of a process of obtaining paired-end sequencing information when only one type of DNA strand is bound to the surface of a solid support. In this example, a first sequencing primer includes a linkable moiety that may be used to anchor a substantially fully extended complementary strand A' to the surface of the solid support. An example of a process of obtaining paired-end sequencing information when only one type of DNA strand is bound to the surface of a solid support may include, but is not limited to, the following steps.

In one step, Figure 2A shows hybridization of a plurality of first sequencing primers 210 to clonal population 120. In this example, clonal population 120 comprises strand A. Each first sequencing primer 210 includes a linkable moiety 215 that may be anchored covalently or non-covalently to solid support 110 under certain conditions. In one example, linkable moiety 215 may be a biotinylated moiety that may be anchored to a streptavidin coated solid support 110 via a biotin-streptavidin complex. In another example, linkable moiety 215 may be any other suitable functional group that may be anchored covalently or non-covalently to solid support 110. Examples of functional groups that may be used to anchor an oligonucleotide to a solid support are described in "Strategies for Attaching Oligonucleotides to Solid Supports" (Integrated DNA Technologies 2014 (v5)). First sequencing primers 210 may be used to obtain the first read in a sequencing reaction, such as sequencing-by-synthesis (SBS), sequencing-by-ligation, or any other suitable sequencing method.

In another step, Figure 2B shows full extension of first sequencing primers 210 after completion of the first sequencing read. First sequencing primers 210 may be extended in the presence of natural dNTPs and DNA polymerase to generate a plurality of substantially full length DNA strands A' 220. DNA strands A' 220 are the complement to clonal population 120 A strands.

In another step, Figure 2C shows anchoring of the fully extended DNA strands A' 220 to solid support 110 via linkable moieties 215. Clonal population 120 (strand A) have been removed. In one example, clonal population 120 were removed by a linearization step (not shown).

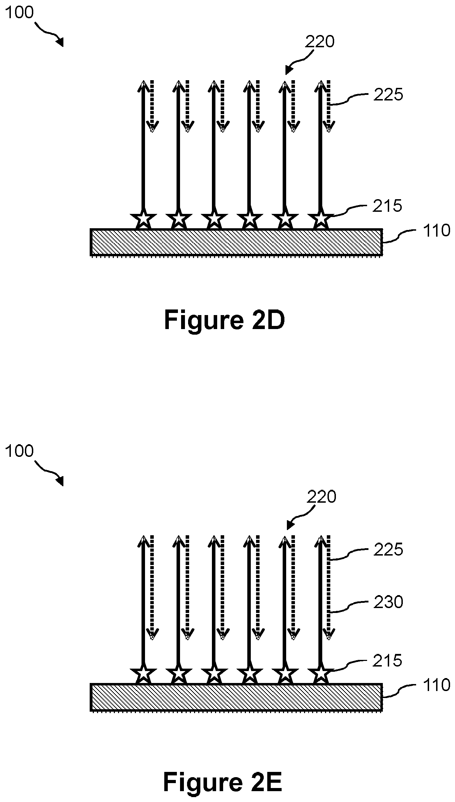

In another step, Figure 2D shows hybridization of a plurality of second sequencing primers 225 to DNA strands A' 220.

In another step, Figure 2E shows sequencing of DNA strands A' 220 by extension of second sequencing primers 225 to obtain a second sequencing read 230 and yield paired-end sequencing information.

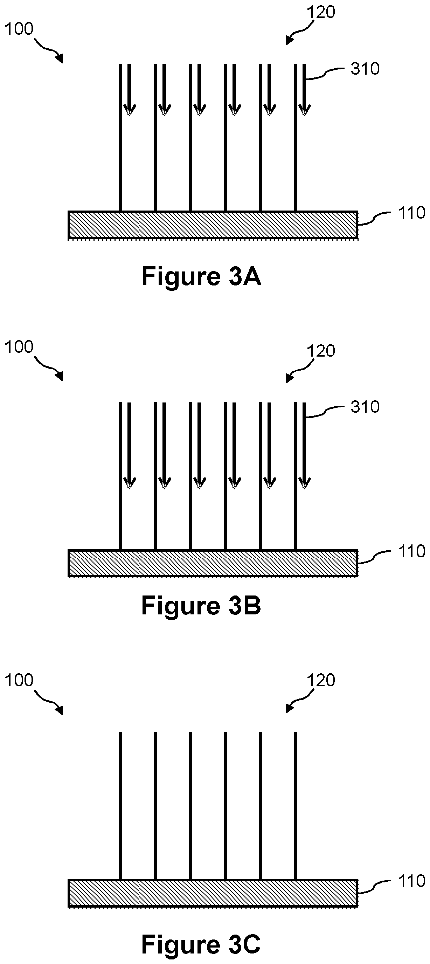

**Figures 3A through 3E** illustrate side views of sequencing substrate 100 of Figure 1B and show another example of a process of obtaining paired-end sequencing information when only one type of DNA strand is bound to the surface of a solid support. In this example, a first sequencing primer that was used to obtain a first sequencing read from strand A is removed and an extension oligonucleotide that includes a linkable moiety is hybridized and used to generate a substantially full length complementary strand A'. The complementary strand A' is anchored to the surface of the solid support via the linkable moiety. In this example, a process of obtaining paired-end sequencing information when only one type of DNA strand is bound to the surface of a solid support may include, but is not limited to, the following steps.

In one step, Figure 3A shows hybridization of a plurality of first sequencing primers 310 to clonal population 120. In this example, clonal population 120 comprises strand A.

In another step, Figure 3B shows extension of first sequencing primers 310 to obtain a first sequencing read in a sequencing reaction, such as SBS, sequencing-by-ligation, or any other suitable sequencing method.

In another step, Figure 3C shows removal of first sequencing primers 310 and the extended first read after completion of the first read sequencing reaction.

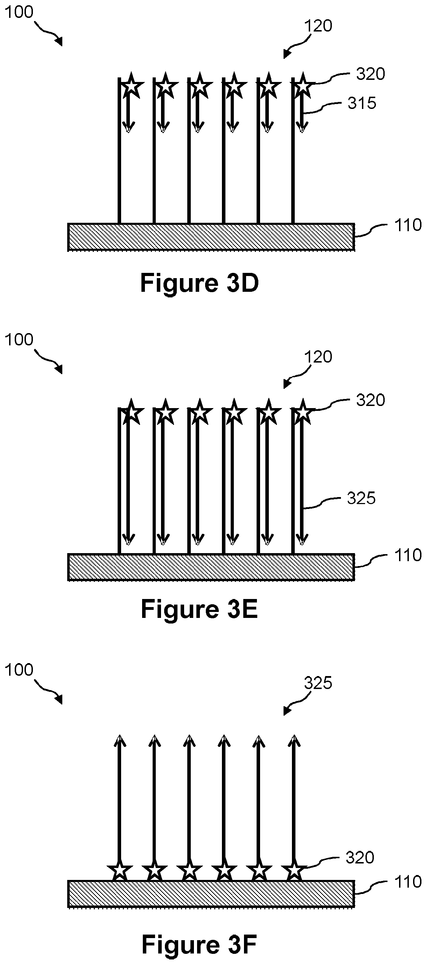

In another step, Figure 3D shows hybridization of a plurality extension oligonucleotides 315 to clonal population 120 (A strands). Each extension oligonucleotide 315 includes a linkable moiety 320 that may be anchored (linked) covalently or non-covalently to solid support 110 under certain conditions.

In another step, Figure 3E shows extension of extension oligonucleotides 315. Extension oligonucleotides 315 can be extended in the presence of dNTPs and a DNA polymerase to generate a plurality of substantially full length DNA strands A' 325. DNA strands A' 325 are the complement to clonal population 120 A strands.

In another step, Figure 3F shows DNA strands A' 325 anchored to the surface of solid support 110 via linkable moieties 320. Clonal population 120 (A strands) have been removed. In one example, clonal population 120 may be removed by a linearization step (not shown).

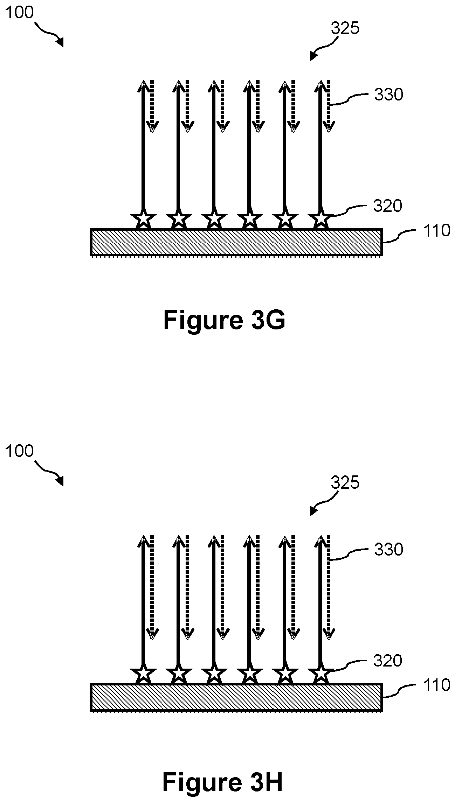

In another step, Figure 3G shows hybridization of a plurality of second sequencing primers 330 to full length DNA strands A' 325 that are anchored via linkable moieties 320 on the surface of solid support 110.

In another step, Figure 3H shows extension of sequencing primers 330 to obtain a second sequencing read. The first sequencing read (Figure 3B) and the second sequencing read provide paired-end sequencing information.

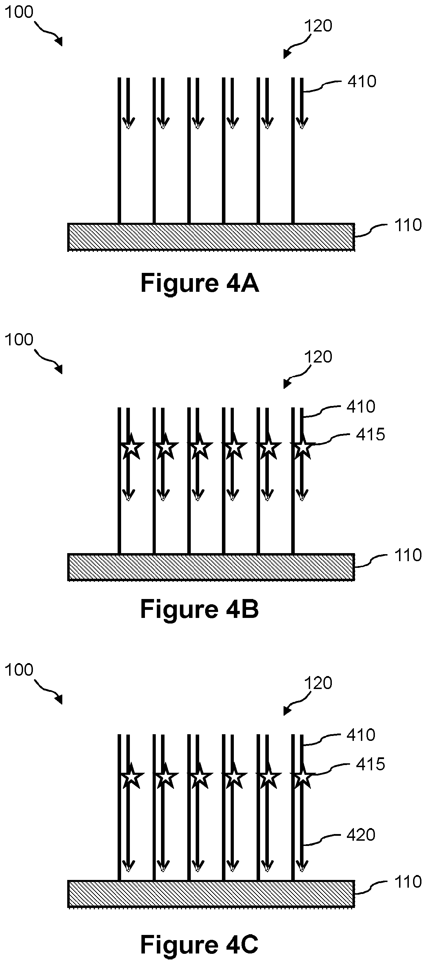

**Figures 4A through 4E** illustrate side views of sequencing substrate 100 of Figure 1B and show yet another example of a process of obtaining paired-end sequencing information when only one type of DNA strand is bound to the surface of a solid support. In this example, modified nucleotides that include a linkable moiety are incorporated in the most 5' portion of the complementary strand A'. In another example (not shown), nucleotides with linkable moieties may be incorporated at a distance further away from the 5' end. The location may depend on insert size and read length. For example, if the insert is very long, it is possible to incorporate modified nucleotides in a substantially central portion, since the reads would not reach the central region of the insert.

In certain embodiments, it may be desirable to include the modified nucleotides near the end of the extension (3' end), since this may improve retention of nucleic acid strands (particularly in cases where sequencing conditions may cause breakage). One method of positioning nucleotides at the 3' end comprises incorporating natural dNTPs to produce the full length strand A', and then adding a polymerase having 3'-5' exonuclease activity in the presence of the modified nucleotides, such that the modified nucleotides can be incorporated at the very 3' end.

In the example illustrated in Figures 4A-4E, a process of obtaining paired-end sequencing information when only one type of DNA strand is bound to a solid surface may include, but is not limited to, the following steps.

In one step, Figure 4A shows hybridization of a plurality of first sequencing primers 410 to clonal population 120. In this example, clonal population 120 comprises strand A.

In another step, Figure 4B shows extension of first sequencing primers 410 and incorporation of modified nucleotides that include a linkable moiety 415 in a sequencing reaction, such as SBS, sequencing-by-ligation, or any other suitable sequencing method. Modified nucleotides that include linkable moieties 415 may be incorporated, for example, during the first few cycles of sequencing. Subsequent rounds of sequencing may be performed with nucleotides that do not include linkable moiety 415.

In another step, Figure 4C shows full extension of first sequencing primers 410 after completion of the first sequencing read. First sequencing primers 410 may be fully extended in the presence of natural dNTPs (e.g., they do not include a linkable moiety) and DNA polymerse to generate a plurality of substantially full length DNA strands A' 420. DNA strands A' 420 are the complement to clonal population 120 A strands.

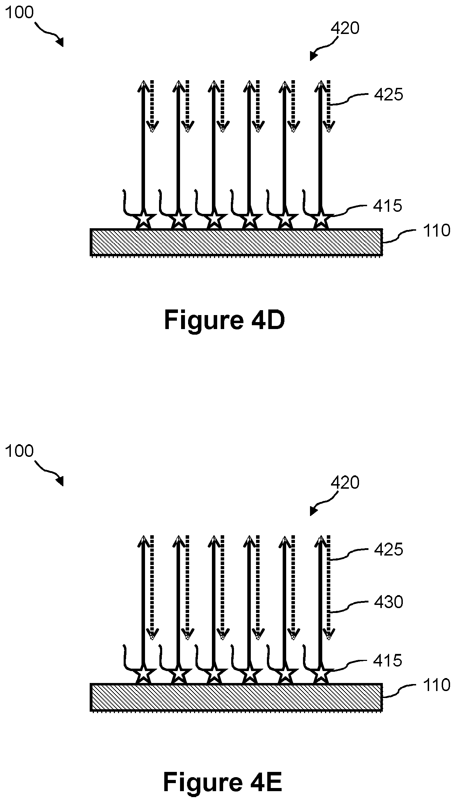

In another step, Figure 4D shows activation of linkable moiety 415 and subsequent linking of DNA strands A' 420 to the surface of solid support 110. Linkable moiety 415 may, for example, be activated by chemical, physical, or enzymatic methods. Because linkable moiety 415 requires an activation step, attachment of free modified nucleotides and saturation of the surface binding sites on the surface of solid support 110 is prevented. Clonal population 120 (A strands) may now be removed. DNA strands A' 420 are now linked to solid substrate 110. A plurality of second sequencing primers 425 are then hybridized to DNA strands A' 420.

In another step, Figure 4E shows extension of second sequencing primers 425 to obtain a second sequencing read 430 and yield paired-end sequencing information.

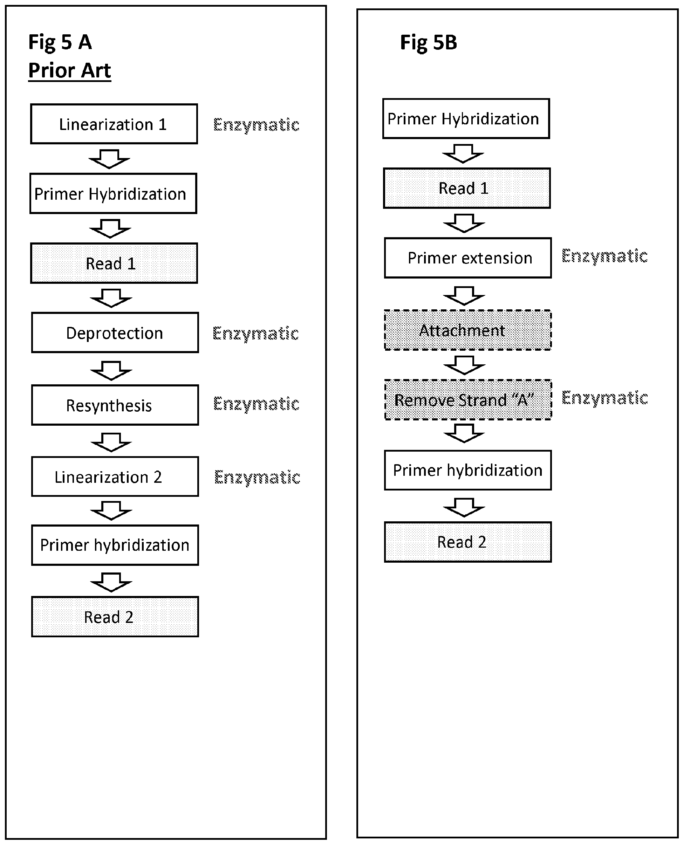

**Figures 5A and 5B** illustrate the simplified method of the present invention (shown in Figure 5B) as compared to prior art methods. The prior art method illustrated in Figure 5A requires a large number of steps to obtain paired-end sequencing information.

It is clear from the method steps illustrated in Figure 5B that the present invention provides an improved, simpler and less time consuming method. In fact, the steps in Figure 5B shown in broken lines ("Attachment" and "Remove Strand A") can be incorporated in cycles of sequencing reactions, further reducing the number of steps and time required for performing the method. The attachment step could be performed by including an activation molecule in the last few cycles of SBS, for example. In this case SBS and attachment are performed simultaneously, thereby eliminating the need for extra time to perform the attachment step as a separate step. Similarly, removal of Strand A by a linearization step could be performed using a linearization enzyme or chemical included in subsequent cycles of SBS. The improved method thus provides significant workflow advantages as compared to prior art protocols.

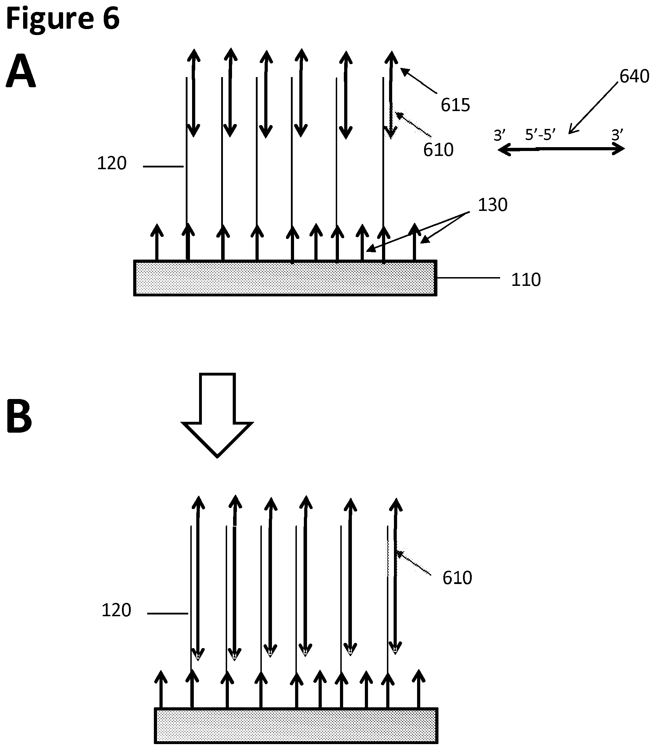

**Figures 6A through 6E** illustrate side views of sequencing substrate 100 of Figure 1B and show an example of a process of obtaining paired-end sequencing information. In this example, the linkable moiety 615 on the first sequencing primer 610 comprises a sequence complementary to a surface primer 130 on the surface of the solid support.

As shown in Figure 6A, the sequence (linkable moiety 615) is attached at its 5' end to the 5' end of the sequencing primer and facing the opposite direction, such that the resulting primer oligonucleotide 640, comprising both the sequencing primer 610 and linkable moiety sequence 615, has two 3' ends.

The primer oligonucleotide 640 may be constructed by chemical linkage of the 5' end of the sequencing primer to the 5' end of the sequence complementary to the surface primer, or via streptavidin/biotin linkages - for example, two biotinylated 5' oligonucleotides bound to streptavidin.

Figure 6A illustrates hybridization of a plurality of these first "double-ended" oligonucleotide primers 640 via hybridization of the sequencing primer component 610 to the clonal population 120. In this example, clonal population 120 comprises strand A. Each strand A is anchored to the solid support 110 by a surface primer 130 on the surface of the solid support. As shown, a number of surface primers 130 remain available for hybridization following amplification.

Typically a large number of surface primers 130 remain available for hybridization. In certain embodiments, however, the reaction conditions for the hybridization step may be optimized to promote hybridization of the oligonucleotide primers 640 to the surface primers 130. For example, higher temperature during hybridization promotes hybridization of the sequencing primer component 610 to the associated site on strand A, as the sequencing primer site has a higher melting temperature than the shorter surface primer sequences 130.

Under certain conditions, oligonucleotide primers 640 may hybridise to surface primers 130 prior to hybridization of the sequencing primer component 610 to an associated site on a strand A. Any oligonucleotide primers 640 that hybridise to surface primers 130 but which fail to also hybridise to strand A will not extend during the sequencing step and may be subsequently removed, for example using heating step.

Similarly, a strand displacing polymerase may be used in the extension step to ensure that any undesirable hybridization of the linkable moiety sequence 615 to part of the molecule being sequenced is corrected during extension, such that the sequence 615 is separated from the nucleic acid strand and therefore free to hybridize to an available surface primer 130.

In one embodiment, undesirable binding of the linkable moiety 615 to surface primers 130 and/or clonal population sequences is reduced by providing additional surface primers having a sequence complementary to the linkable moiety 615, but which sequence differs from that of surface primers 130 linking the clonal population strand A to the solid support.

As shown in Figure 6B, first sequencing primers 610, having the linkable moiety sequence 615 attached to the 5' end may be used to obtain the first read in a sequencing reaction, such as sequencing-by-synthesis (SBS), sequencing-by-ligation, or any other suitable sequencing method.

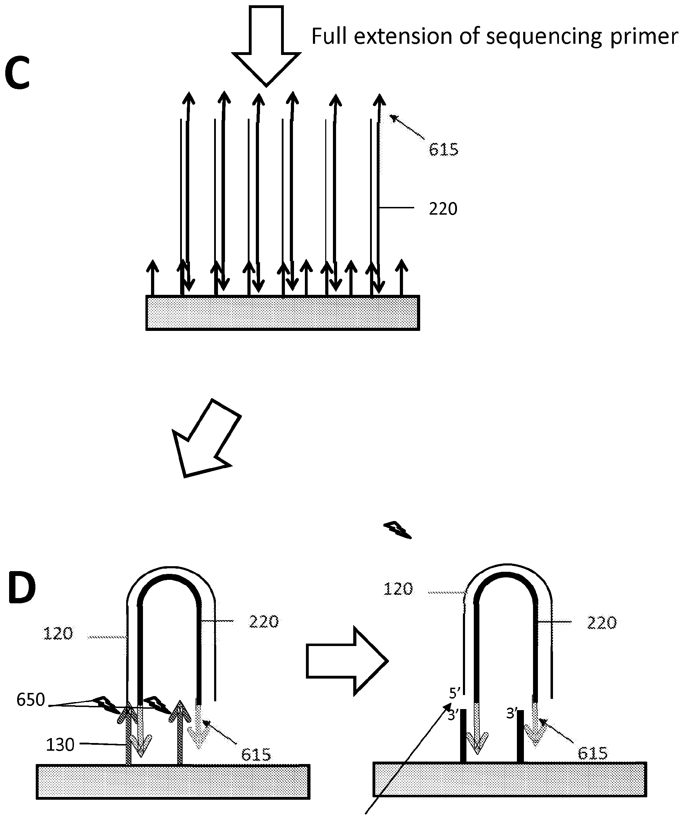

Figure 6C shows full extension of first sequencing primers 610 after completion of the first sequencing read. First sequencing primers 610 may be extended in the presence of dNTPs and DNA polymerase to generate a plurality of substantially full length DNA strands A' 220. DNA strands A' 220 are the complement to clonal population 120 A strands.

As illustrated in Figure 6D, the linkable moiety sequence 615 on the oligonucleotide primer 640 is hybridized to an available surface primer 130 on the solid support, forming a bridge as shown and anchoring the A' strand to the solid support. A linearization step (indicated at 650) linearises the surface primer 130 near its 3' end to generate a 5' end in the strand A 120. Strand A may then be removed using a 5'-3' exonuclease.

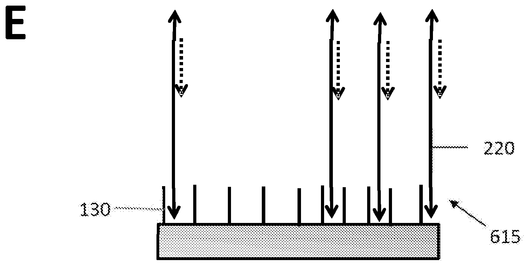

The fully extended DNA strands A' 220 are thus anchored to solid support 110 via hybridization of linkable moieties 615 to the surface primers 130. Clonal population 120 (strand A) have been removed. Figure 6E shows hybridization of a plurality of second sequencing primers 225 to DNA strands A' 220.

DNA strands A' 220 are then sequenced by extension of second sequencing primers 225 to obtain a second sequencing read and yield paired-end sequencing information.

The embodiment of the invention illustrated in Figures 6A to 6E provides the advantage that a conventional solid support may be used, without any additional surface chemistry required to anchor strand A' to the surface.

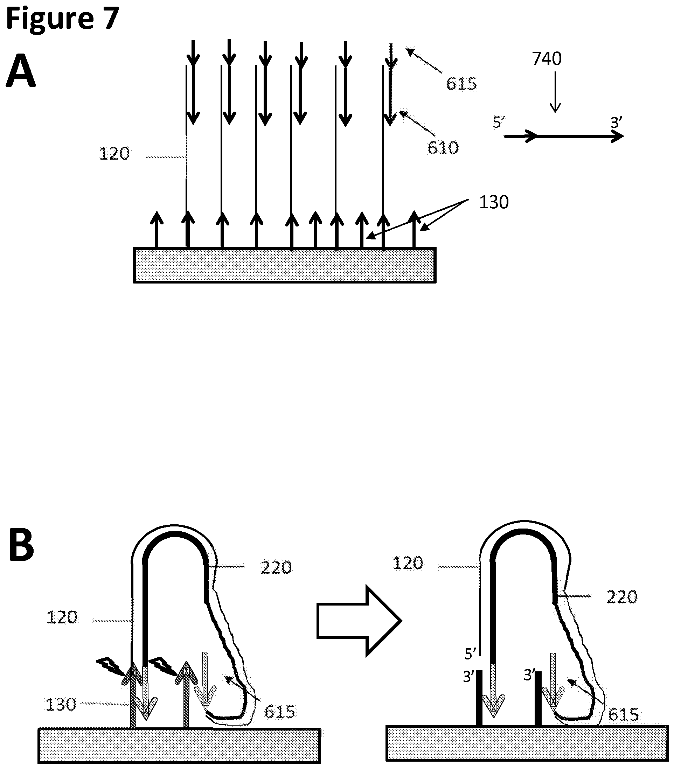

Figures 7A and 7B illustrate an embodiment in which the method illustrated in Figures 6A to 6E may be performed using an alternative oligonucleotide primer 740 comprising a sequence complementary to a the surface primer (linkable moiety 615) attached at its 3' end to the 5' end of a sequencing primer and facing the same direction, such that the resulting primer oligonucleotide 240, comprising both the sequencing primer 610 and linkable moiety sequence 615, has a 5' and a 3' end.

Figure 7B more accurately illustrates the method step shown in and described in relation to Figure 6D, when using the alternative primer oligonucleotide of Figure 7A.

## Claims

1. A method of obtaining paired-end sequencing information in a sequencing reaction comprising the steps of: providing a target nucleic acid immobilized on a solid support; determining the sequence of the target nucleic acid by generating a complementary nucleic acid strand, the complementary nucleic acid strand comprising at least one linkable moiety suitable for immobilization; determining the sequence of the complementary nucleic acid strand; and comparing the sequences of the target nucleic acid and the complementary nucleic acid strand to provide the paired-end sequencing information.

2. The method of claim 1, wherein the at least one linkable moiety is provided on a first sequencing primer.

3. The method of claim 1 or 2, wherein the at least one linkable moiety is provided on an extension oligonucleotide.

4. The method of any preceding claim, wherein the at least one linkable moiety is provided on a modified nucleotide.

5. The method of any preceding claim, wherein the solid support comprises at least one corresponding linkable moiety for selectively covalently or non-covalently binding the at least one linkable moiety on the complementary nucleic acid strand under predetermined conditions, optionally wherein the at least one corresponding linkable moiety comprises a surface oligonucleotide on the solid support and the at least one linkable moiety comprises a nucleic acid sequence substantially complementary to the surface oligonucleotide, optionally comprising the step of activating the at least one linkable moiety to bind the complementary nucleic acid strand to the solid support.

6. The method of any preceding claim, wherein the method comprises the step of removing the target nucleic acid following the step of determining its sequence.

7. The method of any preceding claim, wherein the step of determining the sequence of the target nucleic acid comprises hybridizing a first sequencing primer to the target nucleic acid and extending the first sequencing primer to obtain a first sequencing read.

8. The method of claim 7, wherein the first sequencing primer is extended to substantially the full length of the target nucleic acid following the first sequencing read.

9. The method of any preceding claim, wherein the step of determining the sequence of the complementary nucleic acid strand comprises hybridizing a second sequencing primer to the complementary nucleic acid strand and extending the second sequencing primer to obtain a second sequencing read.

10. The method of claims 7 to 9, wherein the sequencing read is obtained using sequencing by synthesis or sequencing by ligation.

11. The method of any preceding claim, wherein the step of comparing the sequences of the target nucleic acid and the complementary nucleic acid strand to provide the paired-end sequencing information utilizes an algorithm and/or computer processor.

12. The method of any preceding claim, comprising the pre-step of clonal amplification of the target nucleic acid molecule.

13. A system to obtain paired-end sequencing information according to any of claims 1 to 12 comprising sequencing apparatus having a solid support for immobilizing a target nucleic acid, an inlet and outlet for sequencing reagents, and means for determining the sequence of the target nucleic acid by generating a complementary nucleic acid strand, the complementary nucleic acid strand comprising at least one linkable moiety suitable for immobilization; determining the sequence of the complementary nucleic acid strand; and comparing the sequences of the target nucleic acid and the complementary nucleic acid strand to provide the paired-end sequencing information.

## Patentansprüche

1. Ein Verfahren zum Erhalten von Paired-End-Sequenzierungsinformationen in einer Sequenzierungsreaktion, beinhaltend die folgenden Schritte: Bereitstellen einer auf einem festen Träger immobilisierten Zielnukleinsäure; Bestimmen der Sequenz der Zielnukleinsäure durch Erzeugen eines komplementären Nukleinsäurestrangs, wobei der komplementäre Nukleinsäurestrang mindestens einen zur Immobilisierung geeigneten verknüpfbaren Molekülteil beinhaltet; Bestimmen der Sequenz des komplementären Nukleinsäurestrangs; und Vergleichen der Sequenzen der Zielnukleinsäure und des komplementären Nukleinsäurestrangs, um die Paired-End-Sequenzierungsinformationen bereitzustellen.

2. Verfahren gemäß Anspruch 1, wobei der mindestens eine verknüpfbare Molekülteil an einem ersten Sequenzierungsprimer bereitgestellt wird.

3. Verfahren gemäß Anspruch 1 oder 2, wobei der mindestens eine verknüpfbare Molekülteil an einem Verlängerungsoligonukleotid bereitgestellt wird.

4. Verfahren gemäß einem vorhergehenden Anspruch, wobei der mindestens eine verknüpfbare Molekülteil an einem modifizierten Nukleotid bereitgestellt wird.

5. Verfahren gemäß einem vorhergehenden Anspruch, wobei der feste Träger mindestens einen entsprechenden verknüpfbaren Molekülteil zum selektiven kovalenten oder nichtkovalenten Binden des mindestens einen verknüpfbaren Molekülteils an dem komplementären Nukleinsäurestrang unter vorher festgelegten Bedingungen beinhaltet, wahlweise wobei der mindestens eine entsprechende verknüpfbare Molekülteil ein Oberflächenoligonukleotid auf dem festen Träger beinhaltet und der mindestens eine verknüpfbare Molekülteil eine zu dem Oberflächenoligonukleotid im Wesentlichen komplementäre Nukleinsäuresequenz beinhaltet, wahlweise beinhaltend den Schritt des Aktivierens des mindestens einen verknüpfbaren Molekülteils, um den komplementären Nukleinsäurestrang an den festen Träger zu binden.

6. Verfahren gemäß einem vorhergehenden Anspruch, wobei das Verfahren den Schritt des Entfernens der Zielnukleinsäure nach dem Schritt des Bestimmens ihrer Sequenz beinhaltet.

7. Verfahren gemäß einem vorhergehenden Anspruch, wobei der Schritt des Bestimmens der Sequenz der Zielnukleinsäure das Hybridisieren eines ersten Sequenzierungsprimers an die Zielnukleinsäure und das Verlängern des ersten Sequenzierungsprimers beinhaltet, um einen ersten Sequenzierungs-Read zu erhalten.

8. Verfahren gemäß Anspruch 7, wobei der erste Sequenzierungsprimer nach dem ersten Sequenzierungs-Read zu im Wesentlichen der vollständigen Länge der Zielnukleinsäure verlängert ist.

9. Verfahren gemäß einem vorhergehenden Anspruch, wobei der Schritt des Bestimmens der Sequenz des komplementären Nukleinsäurestrangs das Hybridisieren eines zweiten Sequenzierungsprimers an den komplementären Nukleinsäurestrang und das Verlängern des zweiten Sequenzierungsprimers beinhaltet, um einen zweiten Sequenzierungs-Read zu erhalten.

10. Verfahren gemäß den Ansprüchen 7 bis 9, wobei der Sequenzierungs-Read unter Verwendung von Sequenzierung durch Synthese oder Sequenzierung durch Ligatur erhalten wird.

11. Verfahren gemäß einem vorhergehenden Anspruch, wobei der Schritt des Vergleichens der Sequenzen der Zielnukleinsäure und des komplementären Nukleinsäurestrangs zum Bereitstellen der Paired-End-Sequenzierungsinformationen einen Algorithmus und/oder Computerprozessor nutzt.

12. Verfahren gemäß einem vorhergehenden Anspruch, beinhaltend den Vorschritt der klonalen Amplifikation des Zielnukleinsäuremoleküls.

13. Ein System zum Erhalten von Paired-End-Sequenzierungsinformationen gemäß einem der Ansprüche 1 bis 12, beinhaltend Sequenzierungsgerät mit einem festen Träger zum Immobilisieren einer Zielnukleinsäure, einem Einlass und Auslass für Sequenzierungsreagenzien und Mitteln zum Bestimmen der Sequenz der Zielnukleinsäure durch Erzeugen eines komplementären Nukleinsäurestrangs, wobei der komplementäre Nukleinsäurestrang mindestens einen zur Immobilisierung geeigneten verknüpfbaren Molekülteil beinhaltet; Bestimmen der Sequenz des komplementären Nukleinsäurestrangs; und Vergleichen der Sequenzen der Zielnukleinsäure und des komplementären Nukleinsäurestrangs, um die Paired-End-Sequenzierungsinformationen bereitzustellen.

## Revendications

1. Une méthode d'obtention d'informations de séquençage d'extrémités appariées dans une réaction de séquençage comprenant les étapes de : fourniture d'un acide nucléique cible immobilisé sur un support solide ; détermination de la séquence de l'acide nucléique cible par la génération d'un brin d'acide nucléique complémentaire, le brin d'acide nucléique complémentaire comprenant au moins une partie pouvant être reliée et convenant pour l'immobilisation ; détermination de la séquence du brin d'acide nucléique complémentaire ; et comparaison des séquences de l'acide nucléique cible et du brin d'acide nucléique complémentaire afin de fournir les informations de séquençage d'extrémités appariées.

2. La méthode de la revendication 1, dans laquelle l'au moins une partie pouvant être reliée est fournie sur une première amorce de séquençage.

3. La méthode de la revendication 1 ou de la revendication 2, dans laquelle l'au moins une partie pouvant être reliée est fournie sur un oligonucléotide d'extension.

4. La méthode de n'importe quelle revendication précédente, dans laquelle l'au moins une partie pouvant être reliée est fournie sur un nucléotide modifié.

5. La méthode de n'importe quelle revendication précédente, dans laquelle le support solide comprend au moins une partie pouvant être reliée correspondante pour lier sélectivement de façon covalente ou non covalente l'au moins une partie pouvant être reliée sur le brin d'acide nucléique complémentaire dans des conditions prédéterminées, facultativement dans laquelle l'au moins une partie pouvant être reliée correspondante comprend un oligonucléotide de surface sur le support solide et l'au moins une partie pouvant être reliée comprend une séquence d'acide nucléique substantiellement complémentaire à l'oligonucléotide de surface, comprenant facultativement l'étape d'activation de l'au moins une partie pouvant être reliée afin de lier le brin d'acide nucléique complémentaire au support solide.

6. La méthode de n'importe quelle revendication précédente, la méthode comprenant l'étape d'élimination de l'acide nucléique cible après l'étape de détermination de sa séquence.

7. La méthode de n'importe quelle revendication précédente, dans laquelle l'étape de détermination de la séquence de l'acide nucléique cible comprend l'hybridation d'une première amorce de séquençage à l'acide nucléique cible et l'extension de la première amorce de séquençage afin d'obtenir une première lecture de séquençage.

8. La méthode de la revendication 7, dans laquelle la première amorce de séquençage est étendue sur substantiellement toute la longueur de l'acide nucléique cible après la première lecture de séquençage.

9. La méthode de n'importe quelle revendication précédente, dans laquelle l'étape de détermination de la séquence du brin d'acide nucléique complémentaire comprend l'hybridation d'une deuxième amorce de séquençage au brin d'acide nucléique complémentaire et l'extension de la deuxième amorce de séquençage afin d'obtenir une deuxième lecture de séquençage.

10. La méthode des revendications 7 à 9, dans laquelle la lecture de séquençage est obtenue à l'aide d'un séquençage par synthèse ou d'un séquençage par ligation.

11. La méthode de n'importe quelle revendication précédente, dans laquelle l'étape de comparaison des séquences de l'acide nucléique cible et du brin d'acide nucléique complémentaire afin de fournir les informations de séquençage d'extrémités appariées utilise un algorithme et/ou un processeur informatique.

12. La méthode de n'importe quelle revendication précédente, comprenant la pré-étape d'amplification clonale de la molécule d'acide nucléique cible.

13. Un système d'obtention d'informations de séquençage d'extrémités appariées selon n'importe lesquelles des revendications 1 à 12 comprenant un appareil de séquençage ayant un support solide pour l'immobilisation d'un acide nucléique cible, une entrée et une sortie pour des réactifs de séquençage, et des moyens pour la détermination de la séquence de l'acide nucléique cible par la génération d'un brin d'acide nucléique complémentaire, le brin d'acide nucléique complémentaire comprenant au moins une partie pouvant être reliée et convenant pour l'immobilisation ; la détermination de la séquence du brin d'acide nucléique complémentaire ; et la comparaison des séquences de l'acide nucléique cible et du brin d'acide nucléique complémentaire afin de fournir les informations de séquençage d'extrémités appariées.
